# EUROPEAN PATENT APPLICATION

(11) **EP 3 858 283 A1**
(43) Date of publication of application: **04.08.2021**
(21) Application number: 19866071.4
(22) Date of filing: 29.09.2019
(51) Int. Cl.: A61B 34/37, A61B 17/00

(54) **SURGICAL ROBOT BASED ON BALL AND SOCKET JOINT AND TACTILE FEEDBACK, AND CONTROL DEVICE THEREOF**

(30) Priority: 30.09.2018 CN 201811152893; 30.09.2018 CN 201811152855
(71) Applicant: Sihong Zhengxin Medical Technology Co., Ltd., Suqian, Jiangsu 223900 (CN)
(72) Inventor: ZHENG, Yang, Nanjing, Jiangsu 210004 (CN); ZHENG, Xing, Nanjing, Jiangsu 210004 (CN)
(74) Representative: Gill Jennings & Every LLP
(86) International application number: PCT/CN2019/108930
(87) International publication number: WO 2020/063949

(57) **Abstract**

A surgical robot based on a ball-and-socket joint and tactile feedback is provided. The ball-and-socket joint includes a spherical member and a joint socket. A first roller and a second roller are arranged in the joint socket to drive a three-dimensional rotation of the spherical member in the joint socket. A sensor is arranged in the joint socket to sense the three-dimensional rotation of the spherical member in the joint socket. A first channel tube, a second channel tube and a surgical instrument are arranged sequentially in a center of the spherical member.

## Description

### TECHNICAL FIELD

The application relates to a surgical robot system, and more particularly to a surgical robot based on a ball-and-socket joint and tactile feedback and a control device thereof.

### BACKGROUND

With the development of technology, surgical robots have been gradually used in the minimally invasive surgery, during which a surgical instrument enters human body through a small incision fixed on the surface to complete the operation. In view of the constraint of the incision and the operation safety, the surgical instrument is required to perform a fixed-point motion at the incision. Currently, the fixed-point motion of the surgical instrument is achieved mainly through the following three manners.

### 1. Passive joint

The movement of the surgical instrument around the incision is realized indirectly through the movement of a front-end joint of a kinematic chain. During the movement, a reaction force of the body surface against the surgical instrument ensures the fixed-point movement. This method has high safety, but the posture of the surgical instrument is hard to adjust during the surgery.

### 2. Mechanical structure

The fixed-point motion is achieved based on movement characteristics of a mechanical structure. The feasible mechanical structures are listed as follows.

### 1) Arc-track mechanism

As long as the incision on the body surface is located at the center of the arc track, the surgical instrument can perform the fixed-point motion. Nevertheless, this mechanism still suffers a driving problem.

### 2) Shaft-drive mechanism

The fixed-point motion can be made as long as the incision on the body surface is located on a drive axis. However, the fixed-point motion is only achieved in one swing direction.

### 3) Parallel four-bar mechanism

Motions of two parallel four-bars are superimposed to achieve the fixed-point motion, but the mechanism requires high processing accuracy and is difficult to operate. In addition, the mechanism is too large and not convenient to surgical operations.

### 3. Active control

The joint motion of the robot is controlled by a software, and the fixed-point motion is achieved only when the number of joints is greater than four. This control method requires a long motion chain of the medical robot and a large number of drive joints, so that the fixed-point motion can be realized by controlling the joint movement through an algorithm.

### SUMMARY

An object of the present disclosure is to provide a surgical robot based on a ball-and-socket joint and tactile feedback and a control device thereof, so as to overcome the defects in the prior art.

Technical solutions of the present disclosure are described as follows.

In a first aspect, the present discourse provides a surgical robot based on a ball-and-socket joint, wherein the ball-and-socket joint consists of a spherical member and a joint socket; a first drive assembly is provided inside and/or outside the joint socket; a limit part is provided in the joint socket; a through hole is provided in a center of the spherical member; and a channel tube is connected to the spherical member; preferably, a first roller and a second roller configured to drive a three-dimensional rotation of the spherical member in the joint socket are arranged in the joint socket; and the first roller and the second roller are driven by a motor.

In some embodiments, a wall of the channel tube is provided with a motor and a roller; and the roller is engaged with a surgical instrument or a passing tube penetrating through the channel tube.

In some embodiments, a rotary motor configured to drive the channel tube to rotate is provided in the spherical member; the rotating motor is used to drive the channel tube to rotate; the channel tube is provided with a motor and a roller; the roller is arranged in the spherical member; and the roller is configured to drive a pipeline penetrating through the channel tube to move forward and backward along an inner wall of the channel tube through a gap on the channel tube.

In some embodiment, the channel tube comprises a first channel tube and a second channel tube;
wherein the first channel tube is directly or indirectly fixedly connected to the spherical member; the first channel tube is configured to drive a penetrating object to rotate along an inner wall of the channel tube; a sensing assembly arranged on the first channel tube is configured to sense a rotational displacement of the penetrating object along the inner wall of the channel tube; and
the second channel tube penetrates into the first channel tube or the spherical member;
a second drive assembly arranged on the second channel tube is configured to drive the penetrating object to move forward and backward the inner wall of the channel tube respectively; and a sensing assembly arranged on the second channel tube is configured to senses a forward-and-backward motion of the penetrating object along the inner wall of the channel tube.

In some embodiments, the joint socket is arranged in a joint motion frame through a spring; and the joint motion frame is connected to a fixed of the surgical robot.

In some embodiments, the joint socket is arranged in the joint motion frame; an outer wall of the joint socket is provided with a joint shell; a border of the joint motion frame is provided with a telescopic part; the telescopic part is slidably connected to the joint shell; the telescopic part is connected to the first drive assembly; and the telescopic part is configured to generate a telescopic movement under a drive of the first drive assembly.

In some embodiments, the joint motion frame comprises a hard outer frame; and a fixed part is provided on the outer frame to connect with the telescopic part; preferably, the fixed part is a sleeve tube fixed on the border of the joint motion frame; an angle between an axis of the sleeve tube and the border of the joint motion frame is 90°; and preferably, the telescopic part is a straight rod engaged with the sleeve tube.

In some embodiments, an angle between the border of the joint motion frame and a movement direction of the telescopic element arranged on the border is 90°.

In some embodiments, a smooth plane is arranged at where the joint shell is connected to the telescopic part; the telescopic part is provided with a sliding block engaged with the smooth plane; and preferably, a lubricating coating is provided on the smooth plane.

In some embodiments, the joint shell is provided with a straight sliding rail; and the telescopic part is provided with a sliding block engaged with the straight sliding rail.

In some embodiments, the surgical robot further comprises a sterile sleeve; the ball-and-socket joint is sleeved in a sterile sleeve; a sterile tube is pierced in the spherical member; and two ends of the sterile tube are tightly connected to two ends of the sterile sleeve, so as to form a sterile channel in the sterile tub for a surgical instrument to enter and exit.

In a second aspect, the present discourse provides a control device for a surgical robot based on a ball-and-socket joint, comprising:
a first ball-and-socket joint; and
a second ball-and-socket joint;
wherein the first ball-and-socket joint comprises a first spherical member and a first joint socket; the second ball-and-socket joint comprises a second spherical member and a second joint socket; a pull rod and a channel tube are connected the first spherical; the pull rod and the channel tube are provided with a forward sensor and the rotation sensor configured to sense a relative displacement of the first spherical member and the second spherical member; the second spherical member is provided with a trackball configured to collect a three-dimensional rotation of the second joint socket with respect to the second spherical member; a sensing base of the trackball is arranged in the first spherical member; and a roller and a rotary encoder are arranged in the first joint socket to collect a three-dimensional rotation of the first spherical member with respect to the first joint socket.

An operator uses the control device to remotely control a mechanical arm of the surgical robot to perform rotation, forward, and pitch movements. The second ball-and-socket joint at a top of a control rod is held to control a rotation of a working-end wrist of the surgical instrument mounted on the mechanical arm. A finger cot and a pressure receptor are provided on the second ball-and-socket joint. A closing of the finger cot causes a spring to deform, which drives a cable to generate an electrical signal to control a closing of an end of the surgical instrument.

In a third aspect, the present disclosure further provides a mask controller or foot controller based on a ball-and-socket joint, which combines a ball-and-socket joint with a mask or a foot pedal; a roller and a rotating sensor are arranged in a joint socket to collect a three-dimensional rotation of at spherical member with respect to the first joint socket; a sensor configured for collecting a rotation of a channel tube is arranged in a spherical member; a push-pull rod is arranged in the channel tube; a forward sensor configured for collecting a relative displacement of the push-pull rod and the channel tube is arranged on the channel tube; a first limit part is arranged on the channel tube, such that the channel tube only rotates in the spherical member but cannot move forward or backward; and a second limit part is arranged between the push-pull rod and the channel tube, such that the push-pull rod moves forward and backward in the channel tube but cannot rotate, which was controlled by the tongue and/or feet of the operator.

The beneficial effects of the present disclosure are described as follows.
1. The present discourse provides a surgical robot based on a ball-and-socket joint, which is simple and portable. A single ball-and-socket joint replaces a complex robotic arm. Each ball-and-socket joint can be individually packaged and transported, and is small and light, being easy to carry and assemble. The surgical robot can also be used rescue, aerospace and aviation.
2. The surgical robot is convenient for mass production. Since a control device and an executive device have the same structure, they can be replaced with each other when in use, and can share the same production line, the same packaging and the same storage during production. Damaged components can be replaced with new components, and the control device and the executive device can also be used as a disposable consumable.
3. The surgical robot is convenient for simultaneous control of hands, mouth and feet. This system allows the surgeon to operate with both hands while freely controlling other robotic arms from his lips, tongue and feet, which is equivalent to adding a few more hands, reducing a doctor or assistant doctor, and avoiding communication obstacles between two people.
4. The surgical robot has dimple structure, and is easy to install and debug. The system is simple and has few components to be controlled. The surgical robot has few failures, and the maintenance thereof is easy. The surgical robot is economical, reducing the economic burden of patients.
5. The surgical robot has flexible layout. Traditional surgical robots only have 4 or less robotic arms. By contrast, the number of the surgical robots of the disclosure can be set according to the actual need to enable multiple surgeons to perform multiple operations at the same time.
6. The surgical robot has a tactile feedback system.

### BRIEF DESCRIPTION OF THE DRAWINGS

The objects, technical solutions and beneficial effects of this disclosure will be further described clearly and completely below with reference to the accompany drawings and embodiments. Presented herein are merely some embodiments of this disclosure. The components described in the accompany drawings can be arranged and designed in different configurations. The embodiments are illustrative, and are not intended to limit the scope of the disclosure. All other embodiments made by those skilled in the art without departing from the spirit of the disclosure should fall within the scope of the disclosure.
FIG 1 schematically depicts a using state of a robot based on a ball-and-socket joint in accordance with Embodiment 1 of the present disclosure;
FIG 2 schematically depicts a structure of a ball-and-socket joint in accordance with Embodiment 1 of the present disclosure;
FIG 3 schematically depicts a structure of a drive assembly in accordance with Embodiment 1 of the present disclosure;
FIG. 4 schematically depicts a structure of a sensing assembly in accordance with Embodiment 1 of the present disclosure;
FIG 5 schematically depicts a structure of a second channel tube configured to allow a penetrating object to move forward and backward in accordance with Embodiment 1 of the present disclosure;
FIG. 6 schematically depicts a structure of a first channel tube configured to allow the penetrating object to rotate in accordance with Embodiment 1 of the present disclosure;
FIG 7 schematically depicts a combination of the first channel tube and the second channel tube in accordance with Embodiment 1 of the present disclosure;
FIG. 8 schematically depicts a combination of the first channel tube, the second channel tube and the ball-and-socket joint in accordance with Embodiment 1 of the present disclosure;
FIG. 9 schematically depicts a structure of a support assembly of the ball-and-socket joint in accordance with Embodiment 1 of the present disclosure;
FIG. 10 schematically illustrates a principle of a deflection of the ball-and-socket joint in the support assembly in accordance with Embodiment 1 of the present disclosure;
FIG. 11 schematically depicts a whole structure of a control platform;
FIG. 12 schematically depicts a structure of a ball-shaped member equipped with a control device therein in accordance with Embodiment 2 of the present disclosure;
FIG 13 schematically depicts a whole structure of the ball-shaped member equipped with the control device therein in accordance with Embodiment 2 of the present disclosure;
FIG. 14 schematically depicts a structure a control device arranged on a mask in a miniaturized manner in accordance with Embodiment 4 of the present disclosure;
FIG. 15 schematically depicts a structure a control device arranged on a food in accordance with Embodiment 5 of the present disclosure;
FIG 16 schematically depicts a structure a control device arranged on a food petal in accordance with Embodiment 5 of the present disclosure;
FIG. 17 schematically depicts a structure of a robot based on a ball-and-socket joint in accordance with Embodiment 7 of the present disclosure;
FIG 18 schematically depicts a structure of a drive assembly in accordance with Embodiment 7 of the present disclosure;
FIG 19 schematically depicts a structure of a puncture channel tube in an abdominal cavity in accordance with Embodiment 7 of the present disclosure; and
FIG. 20 schematically illustrates a principle of a deflection in accordance with Embodiment 7 of the present disclosure.

In the drawings: 1, ball-and-socket joint; 2, channel tube; 3, support assembly; 4, mechanical arm; 5, drive assembly; 6, sensing assembly; 7, control device; 8, executive device; 9, telescopic rod;
11, spherical member; 12, through hole; 13, joint shell; 14, anti-rotation crossbeam; 15, anti-rotation fixed frame;
21, threaded channel tube; 22, gap; 23, bellmouth; 24, socket; 25, tapered tube neck; 26, transition tube; 27, drive seat; 28, fixed nut;
31, joint motion frame; 32, suspension spring; 33, support rod; 34, support frame; 35, first fixed frame; 36, second fixed frame; 37, connecting rod; 38, sliding rail; 39, sliding block;
41, skin layer; 42, muscle layer; 43, puncture point; 44, puncture channel tube; 45, spherical rod head;
51, drive member; 52, drive motor; 53, transmission device and circuit board; 54, third fixed frame;
61, sensing member; 62, sensor; 63, sensing roller; 64, sensing magnetic ring; 65, Hall sensor and circuit board; 66, housing;
71, hand controller; 72, three-dimensional display; 73, support frame of controller; 74, elbow support; 75, seat;
81, oral controller; 82, mask; 83, anti-pressure rubber ring; 84, headband; 85, miniature ball-and-socket joint controller; 86, miniature drive seat; 87, miniature control rod; 88, hemispherical rod head; 89, waterproof elastic membrane;
91, foot controller; 92, foot support; 93, control rod; 94, toe; 95, elastic sling; 96, pedal; 97, shoe upper; and 98, button.

### DETAILED DESCRIPTION OF EMBODIMENTS

### Embodiment 1

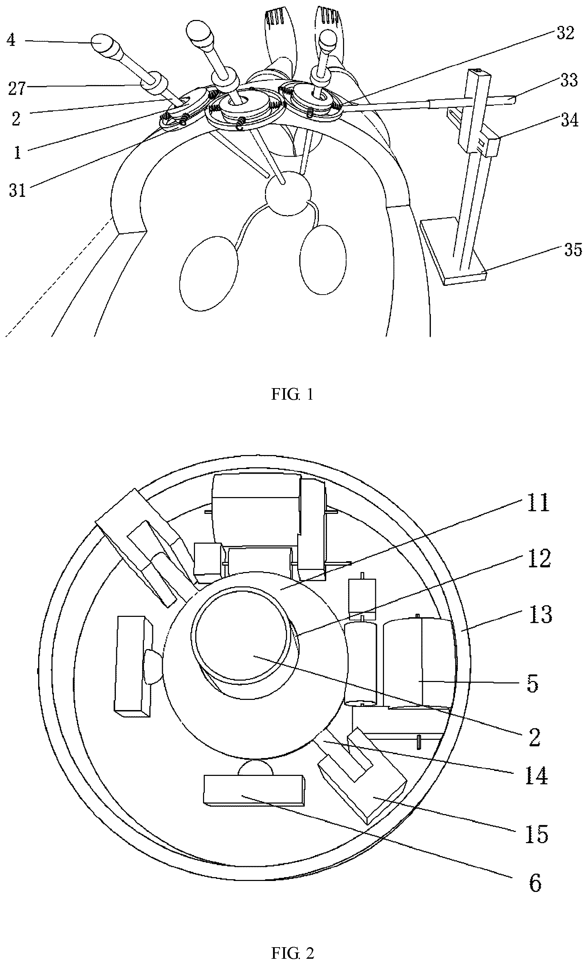

A structure of a robot based on a ball-and-socket joint of the disclosure is shown in FIG. 1. A joint motion frame 31 is fixedly arranged above a patient. A channel tube 2 configured to penetrate a surgical instrument passes through a spherical member 11 in a center of a ball-and-socket joint 1 into an abdominal cavity. A mechanical arm of the robot passes through the channel tube 2 and enters the abdominal cavity. The ball-and-socket joint 1 is fixed in the joint motion frame 31 by a suspension spring 32, and the joint motion frame 31 is fixed on a first fixed frame 35 beside an operating table by a support rod 33 and a support frame 34. The first fixed frame 35 is arranged on a floor of an operating room. In an embodiment, the first fixed frame 35 includes a horizontal rod, a vertical rod and a fixed sliding block along the operating table, and is fixedly connected to the operating table or a ceiling above the patient in a form of a suspended ceiling. An inflated abdominal wall is flat and lacks elasticity, and the robot can also be affixed on the abdominal wall. It should be noted that the disclosure is not limited to this connection mode.

In the robot, the ball-and-socket joint 1 and a support assembly 3 can be sequentially connected to the support rod 33, and a plurality of the ball-and-socket joints 1 can be connected to each other and then connected to the support rod 33, so that the robot can be constructed compactly and smartly.

As shown in FIG. 2, the ball-and-socket joint 1 includes the spherical member 11 and a joint shell 13. A circular opening is provided on the joint shell 13, and a diameter of the circular opening is smaller than a diameter of the spherical member 11. The joint shell 13 is includes two symmetrical parts that are clapped together. The clapped spherical member 11 is arranged in the joint shell 13, and is clamped by two mutually perpendicular drive assemblies 5 and two mutually perpendicular sensing assemblies 6. The drive assemblies 5 and the sensing assemblies 6 are fixedly arranged in the joint shell 13 by fixed clamps. Contact points of the drive assemblies 5 and the sensing assemblies 6 with the spherical member 11 are symmetrically distributed around a center point of the spherical member 11, and are arranged on a plane parallel to a bottom surface of the joint shell 13. Types and positions of the drive assemblies 5 and the sensing assemblies 6 are marked on a surface of the joint shell 13 corresponding to the installation positions. In an embodiment, the joint shell 13 is made of a transparent material, and positions of different components therein can be directly seen.

Anti-rotation crossbeams 14 are symmetrically arranged on the spherical member 11, and are stuck in grooves of anti-rotation fixed frames 15. The grooves of the anti-rotation fixed frames 15 are perpendicular to the bottom surface of the joint shell 13, preventing the spherical member 11 from rotating along a long axis of the channel tube 2 during movement.

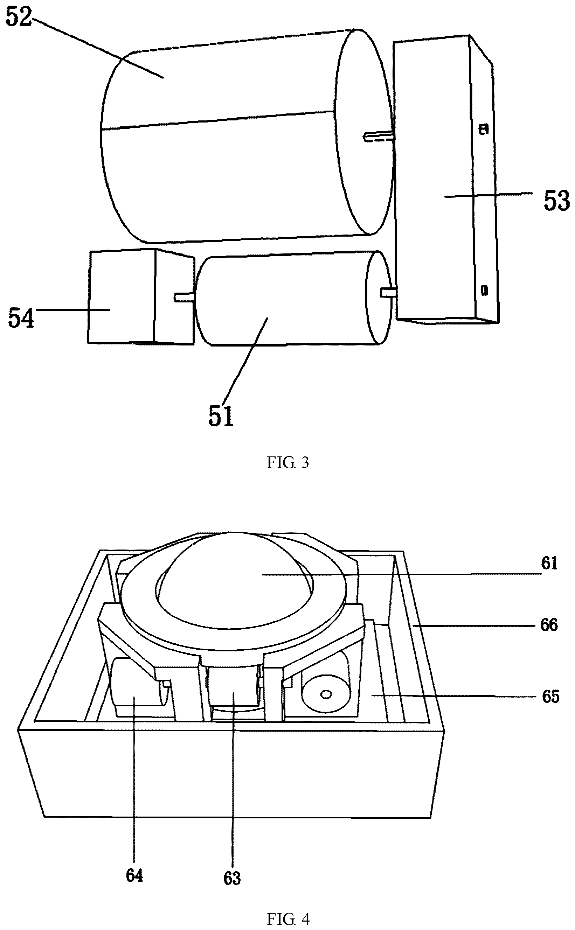

As shown in FIG. 3, the drive assembly 5 is driven by a drive motor 52, and a power is transmitted to a drive member 51 through a transmission device 53. In this embodiment, the drive member 51 has a roller-shaped structure, and is fixedly arranged on the joint shell 13 by a third fixed frame 54. In an embodiment, on the basis of the Embodiment 1, one more drive assembly 5 is added to increase a driving force, and one more sensing assembly 6 is also added at a symmetrical position with respect to the spherical member 11 to sense a movement of the spherical member 11 driven by the newly added drive assembly 5. Contact points of the newly added assemblies with the spherical member 11 are on the same plane as the contact points in the Embodiment 1.

The sensing assembly 6 is in contact with the spherical member 11, which provides support for the spherical member 11 as well as senses the movement of the spherical member 11. A rotation of the spherical member 11 can drive a sensing member 61 on the sensing assembly 6 to rotate accordingly. As shown in FIG. 4, the sensing member 61 in this embodiment has a universal trackball structure. The rotation of the sensing member 61 can drive a sensing roller 63 to rotate, and a sensing magnetic ring 64 arranged on the sensing roller 63 rotates accordingly. A Hall sensor 65 arranged on a circuit board senses a change of a magnetic pole of the sensing magnetic ring 64 and generates a pulse signal, which is sent to a control device 7 of the robot through a signal transmission system.

In an embodiment, the sensing assembly 6 and the drive assembly 5 have the same structure, except that the drive motor 52 is replaced by a rotary encoder. When the drive motor 52 in the drive assembly 5 rotates, the rotary encoder at a corresponding position in the sensing assembly 6 also rotates proportionally.

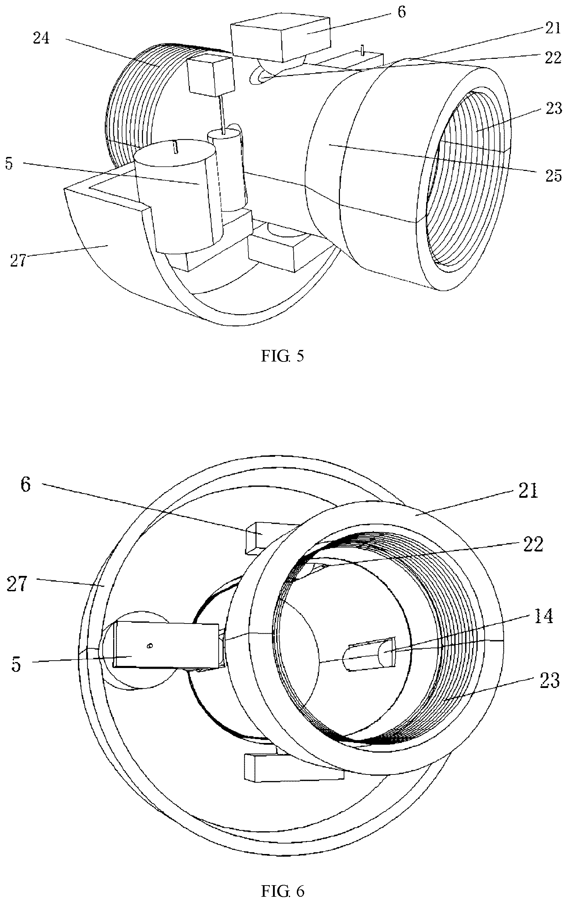

As shown in FIGs. 5-6, the same drive assembly 5 and sensing assembly 6 as those in the ball-and-socket joint 1 are arranged in a drive seat 27, and are fixed by fixed clamps. FIG. 5 shows a second channel tube configured to allow a penetrating object to move forward and backward along an inner wall of the channel tube 2. Driving directions of all the drive members 51 in the drive seat 27 are all parallel to a long axis direction of the second channel tube. FIG. 6 shows a first channel tube configured to allow the penetrating object to rotate along an inner wall of the channel tube 2. Driving directions of all the drive parts 51 in the drive seat 27 are perpendicular to a long axis direction of the first channel tube.

The channel tube 2 passes through the drive seat 27, and is fixedly connected to the drive seat 27. A wall of the channel tube 2 arranged in the drive seat 27 is provided with a gap. The drive member 51 passes through the gap 22 of the channel tube 2 to contact the penetrating object inside the channel tube 2, and drives the penetrating object to move. The sensing member 61 passes through the gap 22 of the channel tube 2 to contact the penetrating object inside the channel tube 2, and sense the movement of the penetrating object. A shape of the gap 22 of the channel tube 2 conforms to shapes of the drive member 51 and the sensing member 61 that need to be passed through.

A threaded bellmouth 23 and a socket 24 are arranged at two ends of the channel tube 2, respectively. An inner diameter of a thread on an inner wall of the bellmouth 23 is the same as an outer diameter of a thread on an outer wall of the socket 24. The socket 24 of one channel tube 2 can be connected to the bellmouth 23 of another channel tube 2. In an embodiment, a socket with a snap ring and a bellmouth with a snap groove are arranged at the two ends of the channel tube 2, respectively. An inner diameter of the snap groove on an inner wall of the socket is the same as an outer diameter of the snap ring on an outer wall of the bellmouth.

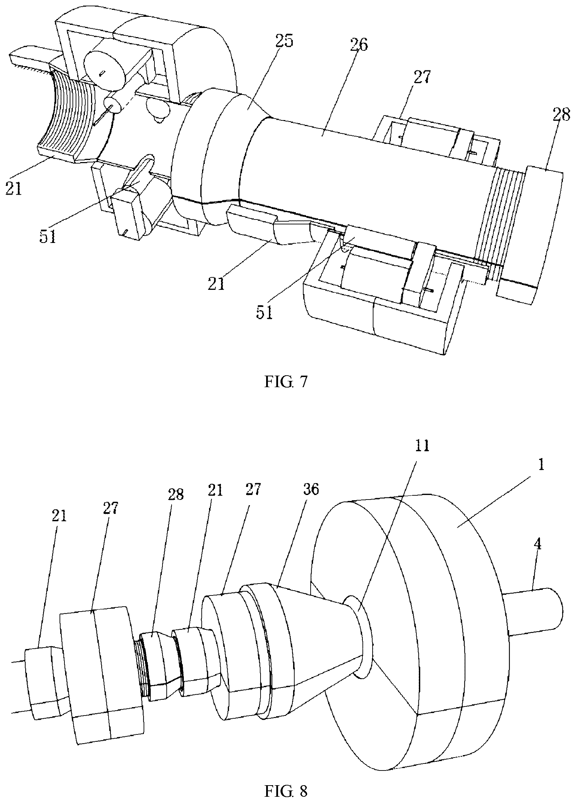

As shown in FIGs. 7-8, a plurality of threaded channel tubes 21 can be connected in series to drive the penetrating object passing through the threaded channel tubes 21 to move. The channel tubes with the same driving direction can be directly connected, and the channel tubes with different driving directions need to be connected through a transition tube 26. An outer diameter of the transition tube 26 is slightly smaller than an inner diameter of the second channel tube, and the transition tube 26 can be inserted into the second channel tube and driven by the drive member 51 to rotate. A tapered tube neck 25 configured for transition is arranged between a bellmouth of the transition tube 26 and a tube body of the transition tube 26. A socket of the transition tube 26 can be connected to a fixed nut 28. An outer diameter of the tapered pipe neck 25 is larger than an inner diameter of a bellmouth of the threaded channel tube 21, and an outer diameter of the fixed nut 28 is larger than an inner diameter of a socket if the threaded channel tube 21, so as to achieve a limit function that the transition tube 26 can rotate but cannot move forward and backward.

The bellmouth of the transition pipe 26 is connected to a socket of the first channel tube, and the first channel tube and the drive seat 27 are driven to rotate together. Since the drive member 51 is closely connected with the penetrating object and generates a friction force therebetween, the penetrating object is also driven to rotate together. In an embodiment, to better drive the penetrating object to rotate, a limit groove parallel to the long axis direction of the channel tube 21 is arranged in the channel tube 21, and a limit slot parallel to the long axis direction of the channel tube 21 is arranged on the penetrating object, such that the penetrating object can rotate accurately while moving forward and backward.

As shown in FIG. 8, a through hole 12 is provided in a center of the spherical member 11, and the spherical member 11 is connected to a second fixed frame 36. The second fixed frame 36 is a tapered pipe communicating with the through hole 12 of the spherical member 11, and can allow a channel tube or a surgical instrument to pass through. An inner diameter of a bellmouth of the second fixed frame 36 is the same as an outer diameter of the drive seat 27 so tightly fasten the driving seat 27. The spherical member 11 drives the drive seat 27 through the second fixed frame 36 to pitch and swing.

An executive device includes the ball-and-socket joint 1, the first channel tube and the second channel tube. The executive device drives the mechanical arm 4 to move forward and backward, rotate, pitch and swing, which is the same as the movement of a laparoscopic instrument operated by human hands.

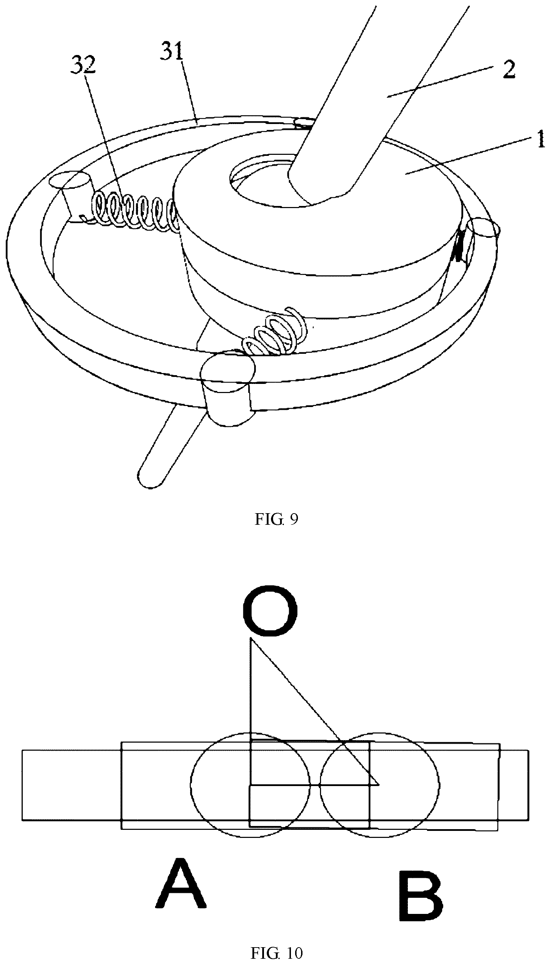

As shown in FIG. 9, during an endoscopic surgery, the mechanical arm 4 enters the abdominal cavity through a puncture point on a skin of a patient, and rotates around the puncture point. Therefore, the endoscopic surgery requires the mechanical arm 4 rotates around a center of the spherical member 11 and the puncture point at the same time. A distance between the puncture point and the center of the spherical member 11 is not less than a radius of the spherical member 11. The rotation of the mechanical arm 4 may cause unnecessary damage to the skin of the patient, and a tension of the skin will also affect an accuracy of the rotation of the spherical member 11. In order to solve this problem, the ball-and-socket joint 1 is connected to the joint motion frame 31 through the suspension spring 32, and the ball-and-socket joint 1 is movable in the joint motion frame 31, such that the mechanical arm 4 can easily rotate around the puncture point.

As shown in FIG. 10, in an embodiment, a central point of the rotation of the mechanical arm 4 is preset at a specific position O on a puncture sheath of the abdominal wall. When a deflection angle of the spherical member 11 is 0 degrees, the ball-and-socket joint 1 is pulled by the suspension spring 32, so that the center of the spherical member 11 is located at a center point A of the joint motion frame 31, and a straight line AO is an axis of the mechanical arm 4 at this time. When the ball-and-socket joint 1 is driven by a motor to rotate, the axis AO of the mechanical arm 4 is deflected to BO with point O as a rotation point, forcing the spherical member 11 to move to point B. In an embodiment, the joint motion frame 31 has a rectangular parallelepiped structure. The three points of ABO form a right-angled triangle. The AO is shorter than the BO. A displacement caused by AO deflection to BO can be compensated after calculation by a host. In another embodiment, the joint motion frame 31 is has hemispherical structure. A length of AO is the same as that of BO or has little difference with that of BO. The displacement caused by AO deflection to BO is 0 or very small, and does not require displacement compensation.

In an embodiment, the robot based on the ball-and-socket joint is used for open surgery. Since there is no skin traction restriction in the open surgery, the ball-and-socket joint 1 can be directly connected to the fixed frame without the joint support assembly 3.

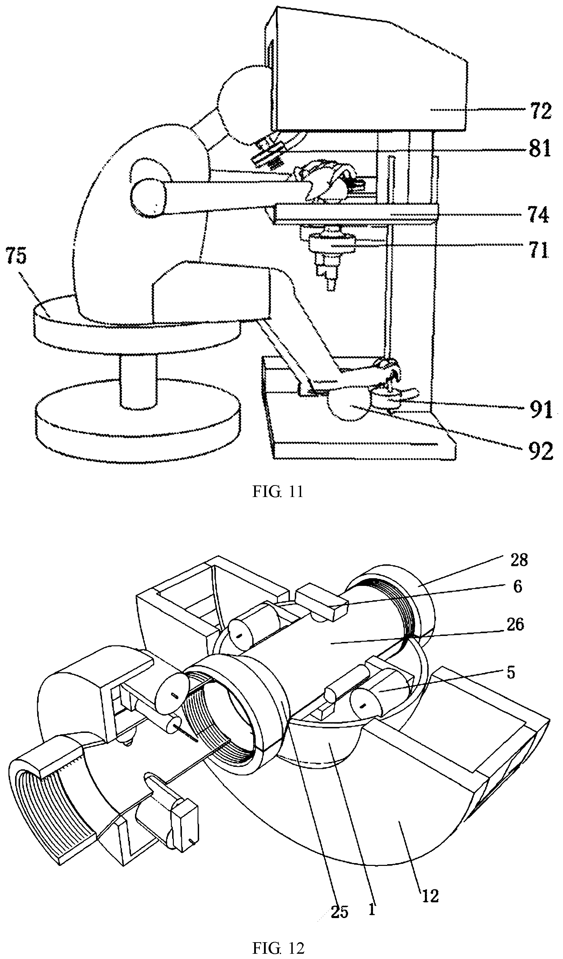

As shown in FIG. 11, hands, mouth and feet of an operator are all provided with the control device 7. The control device 7 has basically the same structure as the executive device, and includes the ball-and-socket joint 1, the channel tube 27 and the support assembly 3. Whereas each driving assembly 5 in the control device 7 is the sensing assembly 6 at a corresponding position of the executive device, and each sensing assembly 6 of the control device 7 is the driving assembly 5 at a corresponding position the executive device. The mechanical arm 4 in the control device is a control rod at a corresponding position the executive device.

A hand movement of the operator is decomposed by a hand controller 71 into forward, backward, rotating, pitching, and swinging movements, which are collected by the sensing assembly 6 and transmitted to the executive device through signal processing, so as to control a movement of the drive assembly 5 at a corresponding position, and drive the mechanical arm 4 to perform the same movement as the hand movement of the operator. At the same time, the movement of the mechanical arm 4 is also collected by the sensing assembly 6 in the executive device, and transmitted to the hand controller 71 through signal processing, so as to control a movement of the corresponding drive assembly 5 and drive the control rod to move. Since the control rod is held by the operator at this time, a force feedback will be given to the operator, producing a tactile sensation of touching the tissue and helping the operator to feel a magnitude of a reaction force.

The robot based on the ball-and-socket joint may have to meet the conflicting needs. In other words, the robot must be placed close to the patient when it works, but at the same time, the medical staff must be able to get close to the patient without obstacles, and when moving, the robot must be above the body of the patient and keep sterile in order to eliminate the infection risk of the patient. In addition, the robot must provide an adequate level of strength, accuracy, and flexibility for locomotion, while equipping with characteristics of small, thin, and light weight. The robot must be arranged in a stable mode while being able to move freely and occupy a small area. Furthermore, the robot should allow the patient and medical staff to move freely in preparation for surgery.

The features of the robot based on the ball-and socket joint are described above in this embodiment. All the robotic arms of the traditional surgical robot based on the ball-and-rocket joint are connected to the robot body, such that the traditional surgical robot is big, and has low mobility. The motion of the traditional surgical robot is complicated. Whereas the robot provided herein is fixed through the support assembly 3 close to the patient, and a length of the mechanical arm 4 above the patient can be limited within the 30 cm. The support assembly (3) can be respectively connected to the fixed support, or connected to one fixed support after being connected to a plurality of fixed frames, such that the robot can move freely with a small occupied area.

Various surgical instruments such as laparoscopic camera, gripper, suction pipe, sucker and actuator can be arranged at an end of the mechanical arm 4.

### Embodiment 2

As shown in FIGs. 12-13, an improvement has been made herein to Embodiment 1. In this embodiment, the spherical member 11 includes the drive assembly 5, the sensing assembly 6 and the transition pipe 26 configured for driving rotation, and is equipped with a function of the drive seat 27. One drive seat 27 configured for driving rotation, one channel tube 21 and the second fixed frame 36. Other structures in this embodiment are the same as those in Embodiment 1.

### Embodiment 3

The structure of the surgical robot in this embodiment is basically the same as that Embodiment 1 except that the executive device is only provided with the drive assembly 5 and the control device 7, is only provided with the sensing assembly 6. The operator cannot feel a force feedback when operating. However, more drive assemblies 5 can be arranged in the executive device to strengthen the driving force.

### Embodiment 4

In this embodiment, the control device 7 of the ball-and-socket joint robot is miniaturized and installed on a mask to form an oral controller 81, which is controlled by the tongue and teeth of operator.

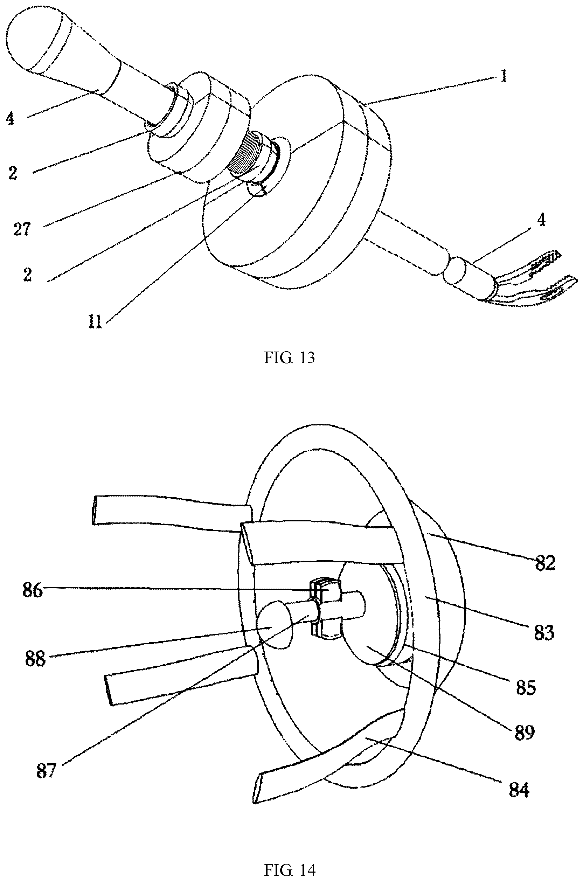

As shown in FIG. 14, a mask body of the mask 82 is tapered, covers the nose and mouth of the operator, and is fixedly connected to the head of the operator by a headband 84. Part of the mask 82 in contact with the human skin is provided with a soft anti-pressure rubber ring 83 to prevent pressure injuries. A protruding part of the mask 82 is equipped with a miniature ball-and-socket joint controller 85, which is wrapped by a waterproof elastic membrane 89, from which a miniature control rod 87 protrudes. The miniature control rod 87 is provided with a miniaturized miniature drive seat 86, and only one sensing assembly 6 and one drive assembly 5 are arranged therein, which is convenient to be made into a long strip, and the first channel tube can be operated by the operator with teeth and lips.

An end of the miniature control rod 87 is a smooth hemispherical rod head 88, which is convenient for the tongue to be rolled up and pulled back or pushed forward. The operator pushes, pulls, and swings the hemispherical club head 88 with the tongue, and uses the teeth and lips to operate the first channel tube, such that the corresponding sensing assembly 6 generates an electrical signal. After real-time translation through a main console circuit, an output signal is transmitted to drive assembly 5 corresponding to the executive device, such that an action of the operator is synchronously reproduced by the mechanical arm 4. The operator can also use the tongue to press a button on the hemispherical head 88 to further control a movement of an instrument at an end of the mechanical arm 4.

### Embodiment 5

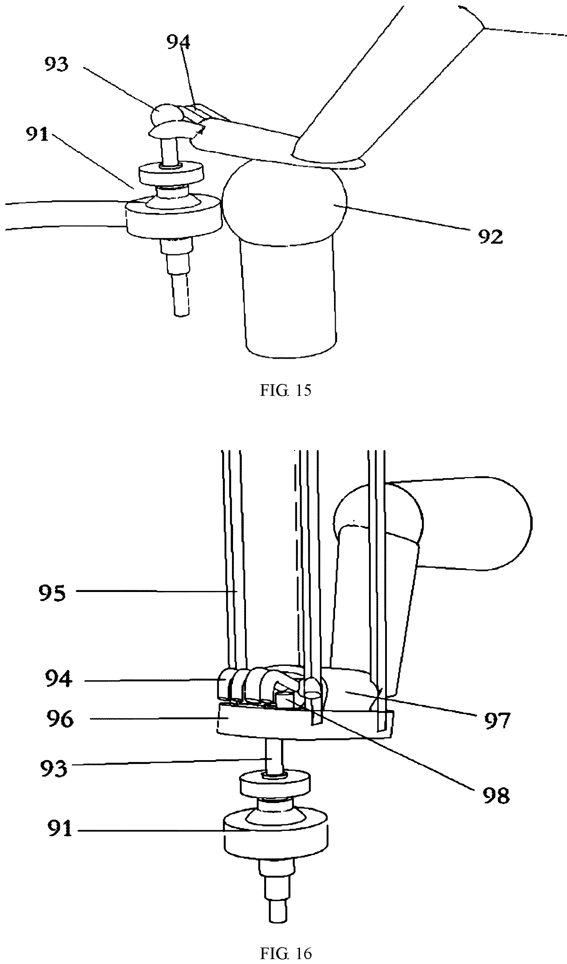

As shown in FIG. 15, a foot controller 91 of the surgical robot is arranged on the console and controlled by the feet of operator.

The operator steps on a ball-shaped foot support 92 with bare feet, and clamps a control rod 93 using toes 94. The operator drives the control rod 93 to rotate, move forward and backward and swing using the feet, so as to make the sense assembly 6 generate an electrical signal. After real-time translation through a main console circuit, an output signal is transmitted to the drive assembly 5 corresponding to the executive device, such that an action of the operator is synchronously reproduced by the mechanical arm 4.

### Embodiment 6

As shown in FIG. 16, this embodiment is similar to Embodiment 5 except that the foot support 92 is a slipper-like structure, which is sleeved on the foot of operator, and is connected to a fixed frame through an elastic sling 95, providing support for the feet and facilitating a free movement of the feet. A pedal 96 is connected to the control rod 93. The pedal 96 is provided with a shoe upper 97, which is convenient for the operator to lift the instep. The pedal 96 is provided with a button 98 for the toes to press.

The operator drives the control rod 93 to rotate, move forward and backward and swing through the feet, so as to make the sense assembly 6 generate an electrical signal. After real-time translation through a main console circuit, an output signal is transmitted to the drive assembly 5 corresponding to the executive device, such that an action of the operator is synchronously reproduced by the mechanical arm 4. The operator can also press the button with the toes to further control a movement of an instrument at an end of the mechanical arm 4.

### Embodiment 7

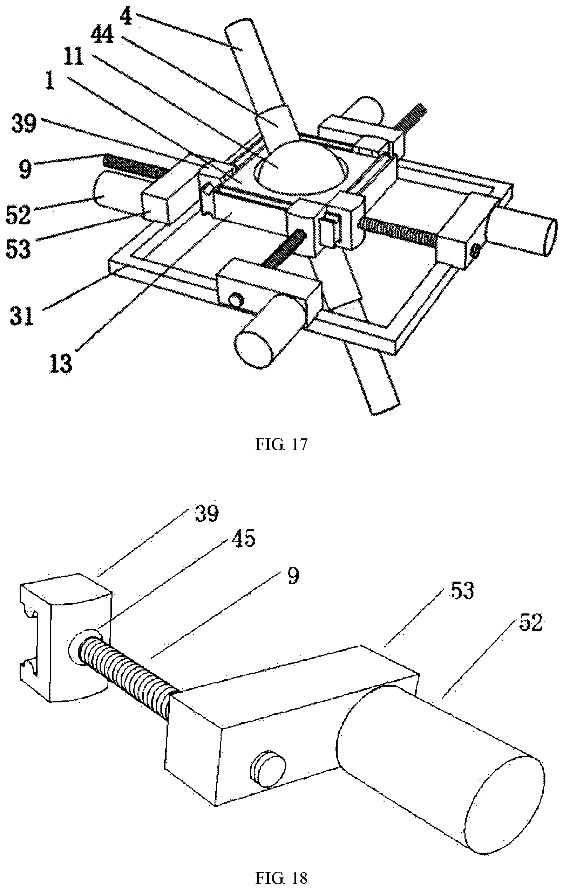

This embodiment is used for laparoscopic surgery, and a laparoscopic surgical robot with universal joints of the present disclosure is used as a mirror-holding arm to replace a surgical assistant. As shown in FIGs. 17-18, the surgical robot includes a ball-and-socket joint 1, a joint shell 13, a drive assembly 5 and a joint motion frame 31. The ball-and-socket joint 1 is arranged in the joint shell 13, and the joint shell 13 is arranged in the joint motion frame 31. The drive assembly 5 is fixedly arranged on a border of the joint motion frame 31. A transmission device 53 is connected to a drive motor 52, and a gear set is connected to a telescopic rod 9 inside the transmission device 53. The telescopic rod 9 is threaded and can move forward and backward through rotating. A spherical head 45 at a top of the telescopic rod 9 is connected to a sliding block 39 through the ball-and-socket joint 1, so as to push the sliding block 39 move forward and backward. The sliding block 39 is slidably connected to a sliding rail 38 on joint shell 13. An angle between the telescopic rod 9 and the border of the joint motion frame 31 on which the telescopic rod 9 is located is 90°, and the telescopic rod 9 is parallel to a plane on which the joint motion frame 31 is located. The joint shell 13 includes two symmetrical parts clapped together. The clapped spherical member 11 is arranged in the joint shell 13. The joint motion frame 31 is erected above an operation area of the patient through a support rod 33 and a support frame 34 fixed on the operating bed.

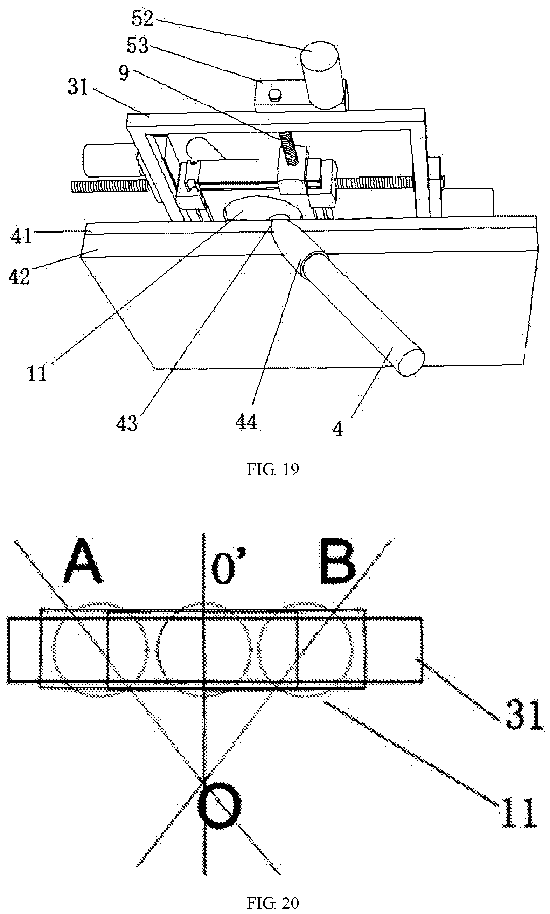

As shown in FIG. 19, a through hole is provided at a center of the spherical member 11, and the puncture channel tube 44 passes through the through hole and enters the abdominal cavity. The puncture channel tube 44 is bound by the abdominal wall of the human body. The main mechanical support is from a skin layer 41 and a muscle layer 42, and other tissues are too weak to support. The surgeon can strengthen the binding force on the puncture channel tube 44 by suturing the skin or muscle at a puncture point 43.

As shown in FIG. 19, when the telescopic rod 9 on a left side of the joint motion frame 31 pushes the ball-and-socket joint 1 to the right, the joint motion frame 31 is fixed by a frame beside the operation table and the ball-and-socket joint 1 drives one end of the puncture channel tube 44 to move to the right. Since the other end of the puncture channel tube 44 is bound at the puncture point 43, the puncture channel tube 44 will deflect, driving the spherical member 11 to rotate inside the joint shell 13. Similarly, when the four telescopic rods 9 on the front, rear, left and right of the joint motion frame 31 push the ball-and-socket joint 1 to an opposite side, the puncture channel tube 44 will be driven to deflect in the abdominal cavity, and at the same time the spherical member 11 is driven to rotate inside the joint shell 13. The mechanical arm 4 enters the abdominal cavity through the puncture channel tube 44 and rotates around the puncture point 43.

As shown in FIG. 20, in an embodiment, a rotation center of the mechanical arm 4 is preset at a specific position O of the puncture channel tube 44. When a deflection angle of the spherical member 11 is 0 degrees, a center of the spherical member 1 is set at a center point O' of the joint motion frame 31. A straight line AO is an axis of the mechanical arm 4 in FIG. 4. When the spherical member 11 outside the body moves from A to B, the axis AO of the mechanical arm 4 is deflected to BO with O point as a rotation point, so as to realize an intra-abdominal deflection.

Since the AO and the BO construct a triangle, they are longer than a vertical line OO', such that the puncture channel tube 44 will produce a small amount of displacement when AO is deflected to OO'. Due to the elasticity of the skin, the puncture channel tube 44 can move up and down in the spherical member 11 with little impact. In another embodiment, the joint motion frame 31 has a hemispherical structure, and the lengths of AO, BO and OO' are the same or have little difference, such that the displacement caused by the deflection of AO to BO is 0 or very small, which further reduces the influence.

### Embodiment 8

A control device connected to an operating platform is connected to a control device controlling the operation of a robotic arm. When in use, the robotic arm is controlled by the operating platform. A command of the operating platform is sent by the control device of the operating platform to the control device of the robot through real-time-clock (RTC) instant messaging. After receiving the command, the control device of the robot will send the command to the robotic arm to preform corresponding actions. The state of the robotic arm is summarized to the control device of the robot, and is sent to the control device of the operating platform through RTC communication. At the same time, the state information of the control device of the operating platform and the state information of the robotic arm are processed and sent to the operating platform, providing a feedback to the staff.

A monitoring device configured for monitoring whether the staff is in place and a display configured for displaying status information of the operating platform and the robot are arranged on the control device of the operating platform. When the monitoring device detects abnormal conditions, it will perform brake or power off according to the monitoring results to ensure the safety of the operation. In an embodiment, the monitoring device includes a 3D motion camera (Kinect) and a foot switch. Only when the 3D motion camera detects that the staff is in place, can the robotic arm perform partial functional operations. When the staff steps on the foot switch, the robotic arm can start to operate. When the staff is not in place and the foot switch is not stepped down, the robotic arm brakes to prevent a movement of the robotic arm due to an operation caused by abnormal factors.

The control device of the robot is provided with the sensing assembly 6 configured for recording a displacement of the mechanical arm 4, so as to record a motion track of the mechanical arm 4. The control device of the robot automatically judges whether the motion track of the mechanical arm 4 meet the operation requirement through the information from the sensing component. When a plurality of mechanical arms 4 are provided, the control device of the robot can monitor whether the movement tracks of the mechanical arms 4 will be interfered with each other, so as to replan a new movement track to ensure the moving safety of the mechanical arms 4. The control device of the robot is also provided with a motor-drive device configured for obtaining a state of the motor and a motor-brake device configured for giving response to a state of the robot. When a dangerous signal is detected, the motor-brake device will brake automatically. The motor-driving device can be used in cooperation with the encoder. When the encoder detects the abnormal movement track of the robotic arm, the encoder can feedback the information to the motor drive device and allow the motor-driving device to drive the motor to start a new working track. The control device of the robot further includes a motor-communication device configured to monitor a feedback between the motor and the motor-drive device and a feedback between the motor-drive device and the robotic arm in real time, and feedback the monitoring information to the staff. When a fault happens, the motor-communication device can perform a brake to ensure the normal operation of the surgical robot according to the monitoring information, or feedback the fault information to the staff, so that the staff can quickly deal with the fault.

A data recording module configured to record a parameter information of the robotic arm and/or a parameter information of the control device of the operating platform is arranged on the operating platform, so as to search the fault information conveniently. Specifically, the data recording module includes a running log and data of a control hand. In an embodiment, the staff operates a handle and sends an operation to the robotic arm through the control device of the operating platform to allow the robotic arm to move. When the robotic arm moves, the information is fed back to the control device of the operating platform through the encoder and the motor-drive device to form a feedback mechanism. If the operating data is inconsistent with the encoder or motor data, the system will automatically adjust. If it can't be adjusted, a fault will happen. The staff needs to check the data of the control hand to find the fault location.

Preferably, an emergency stop switch configured for emergency brake is provided on the control device of the operating platform to perform an emergency brake on the robot when a fault occurs, so as to reduce a loss caused by the fault.

In an embodiment, the control device of the robot and the control device of the operating platform are respectively provided with a first uninterruptible power supply (UPS) and a second UPS to monitor a voltage and protect a circuit. The first UPS and the second UPS can monitor the voltage and stability of a power grid. When power off happens, the first UPS and the second UPS can help the operation continue normally, and feedback a monitoring information to the staff through the control device of the operating platform.

The control device of the robot and the control device of the operating platform are respectively provided with a first power monitor and a second power monitor to monitor a circuit state of each part of the control devices. If the voltage and current of the circuit are within a scope of the preset parameter, the control devices are working normally. When the voltage and current of the circuit exceed the preset parameter in the fault, the control devices immediately perform the brake or power off.

A first indicator light configured for showing a working state of the robotic arm and a second indicator light configured for showing a working state of the operating platform are respectively provided on the control device of the robot and the control device of the operating platform. The indicator lights can present the working state of the robotic arm and the operating platform to the staff intuitively. According to a display of the indicator lights, the staff can quickly find the fault by checking the error code. Specifically, indicating states of the indicator lights include normal, standby, warning, and danger, so that the staff can monitor the use of the robot.

Based on the control devices provided in the above embodiment, a surgical robot is further provided, which include an operating platform, a robotic arm, a control device connected with the operating platform and a control device connected to the robotic arm. The control devices are the same with the above-mentioned the control device. The structure of other parts of the surgical robot is referred to the prior art, and will not be repeated herein.

The above-mentioned control devices provide the surgical robot with a high safety when in use.

As used herein, it should be noted that unless otherwise specified, the terms "setup", "install", "connect" and "link" should be understood in a broad sense. For example, it can be interpreted as fixed connection, replaceable connection or integral connection, or can be interpreted as mechanical connection or electrical connection, or can be interpreted as direct connection or indirect connection through an intermediate medium, or can be interpreted as communication between two elements. For those skilled in the art, the specific meanings of the above terms can be understood according to the corresponding description.

It should be noted that similar reference numerals and letters indicate similar items in the following drawings. Therefore, once a certain item is defined in one drawing, it does not need to be further defined and explained in the subsequent drawings.

As used herein, the orientation or position terms, such as "center", "upper", "lower", "left", "right", "vertical", "horizontal", "inner" and "outer", are intended to illustrate the orientation or positional relationship shown in the drawings. These terms are merely illustrative of this disclosure, and do not indicate or imply that the device or element referred to must have the specified orientation, or must be constructed and operated in the specified orientation. Therefore, these terms should not be interpreted as a limitation to this disclosure. In addition, the terms "first", "second" and "third" are only used for description, and should not be understood to indicate or imply the relative importance. It should be noted that these embodiments are merely preferred embodiments, and are not intended to limit this disclosure. Any variations, modifications, replacements and improvements made without departing from the spirit of the present disclosure should fall within the scope of the disclosure defined by the appended claims.

## Claims

1. A surgical robot based on a ball-and-socket joint, wherein the ball-and-socket joint consists of a spherical member and a joint socket; a first drive assembly is provided inside and/or outside the joint socket; a limit part is provided in the joint socket; a through hole is provided at a center of the spherical member; and the spherical member is connected to a channel tube.

2. The surgical robot of claim 1, wherein the first drive assembly comprises a drive part and a drive motor; and the drive part is directly connected to the drive motor, or is connected to the drive motor through a transmission device.

3. The surgical robot of claim 1, wherein a wall of the channel tube is provided with a drive seat; a second drive assembly is arranged in the drive seat to drive a penetrating object to move forward and backward along the spherical member; the second drive assembly is engaged with the penetrating object; and the penetrating object is a surgical instrument penetrating through the channel tube.

4. The surgical robot of claim 3, wherein a third drive assembly is arranged in the spherical member; and the third drive assembly is configured to drive the penetrating object to rotate along an inner wall of the spherical member.

5. The surgical robot of claim 1, wherein the channel tube penetrates through the spherical member; and the channel tube is detachably connected to the spherical member.

6. The surgical robot of claim 1, wherein the channel tube comprises a first channel tube; the first channel tube is fixedly connected to the spherical member directly, or fixedly connected to the spherical member through a first transition tube; and the first channel tube is provided with a fourth drive assembly to drive a penetrating object to rotate along an inner wall of the spherical member.

7. The surgical robot of claim 6, wherein the channel tube further comprises a second channel tube; the second channel tube directly penetrates into the first channel tube or the spherical member, or penetrates into the first channel tube or the spherical member through a second transition tube; and the second channel tube is provided with a fifth drive assembly to drive the penetrating object to move forward and backward along the inner wall of the spherical member.

8. The surgical robot of claim 1, wherein the ball-and-socket joint is arranged in a joint motion frame through a spring; and the joint motion frame is connected to a fixed support of the surgical robot.

9. The surgical robot of claim 1, wherein the joint socket is arranged in a joint motion frame; an outer wall of the joint socket is provided with a joint shell; a border of the joint motion frame is provided with a telescopic part; the telescopic part is slidably connected to the joint shell; the telescopic part is connected to the first drive assembly; and the telescopic part is configured to generate a telescopic movement under a drive of the first drive assembly.

10. The surgical robot of claim 9, wherein the joint motion frame comprises a hard outer frame; and a fixed part is provided on the outer frame to connect with the telescopic part.

11. The surgical robot of claim 9, wherein an angle between the border of the joint motion frame and a movement direction of the telescopic part arranged on the border is 90°.

12. The surgical robot of claim 9, wherein a smooth plane is arranged at where the joint shell is connected to the telescopic part; and the telescopic part is provided with a sliding block that fits with the smooth plane.

13. The surgical robot of claim 9, wherein the joint shell is provided with a straight sliding rail; and the telescopic part is provided with a sliding block that fits with the straight sliding rail.

14. A surgical system based on a ball-and-socket joint and tactile feedback, comprising:
the surgical robot of claim 1; and
a sensor;
wherein the sensor is provided in the first drive assembly; the sensor is directly connected to the drive part, or connected to the drive part through a transmission device; and the sensor is configured to sense a rotation of the drive part.

15. A control device of a surgical robot based on a ball-and-socket joint, wherein the ball-and-socket joint consists of a spherical member and a joint socket; a sensing assembly is provided inside and/or outside the joint socket; a first limit part is provided in the joint socket; a through hole is provided at a center of the spherical member; the spherical member is connected to a channel tube; and a second limit part is provided at an inner wall of the channel tube.
